# EUROPÄISCHE PATENTANMELDUNG

(11) **EP 1 666 130 A1**
(43) Veröffentlichungstag der Anmeldung: **07.06.2006**
(21) Anmeldenummer: 05024882.2
(22) Anmeldetag: 15.11.2005
(51) Int. Cl.: B01D 71/02, A61K 9/107, B01F 3/08, A23D 7/00

(54) **Verfahren zur Herstellung einer feinteiligen Emulsion aus einer Rohemulsion**

(30) Priorität: 17.11.2004 DE 102004055542
(71) Anmelder: BASF Aktiengesellschaft, 67056 Ludwigshafen (DE)
(72) Erfinder: Danner, Thomas, Dr., 67167 Erpolzheim (DE); Voss, Hartwig, Dr., 67227 Frankenthal (DE); Bauder, Andreas, 68199 Mannheim (DE); Viereck, Sonja, 68163 Mannheim (DE)
(74) Vertreter: Isenbruck, Günter

(57) **Zusammenfassung**

Es wird ein Verfahren zur Herstellung einer feinteiligen Emulsion aus einer Rohemulsion vorgeschlagen, wobei die Rohemulsion durch eine poröse Membran gedrückt wird, das dadurch gekennzeichnet ist, dass die poröse Membran aus zwei oder mehreren übereinanderliegenden Schichten aufgebaut ist, die sich durch den Porendurchmesser unterscheiden.

## Beschreibung

Die vorliegende Erfindung betrifft ein Verfahren zur Herstellung einer feinteiligen Emulsion aus einer Rohemulsion, wobei die Rohemulsion durch eine poröse Membran gedrückt wird.

Emulsionen werden in der Pharma-, Lebensmittel- und Kosmetikindustrie in großem Umfang eingesetzt. Die Eigenschaften von Emulsionen, wie Stabilität und rheologisches Verhalten, werden in besonderem Maße von der Tröpfchengrößenverteilung in der Emulsion beeinflusst. So steigt die Stabilität von ÖI-in-Wasser oder von Wasser-in-Öl-Emulsionen mit enger werdender Tröpfchengrößenverteilung an. Ein besonderer Schwerpunkt bei der Erzeugung von Emulsionen liegt demnach auf der Tröpfchengrößenverteilung und dem mittleren Tröpfchengrößendurchmesser.

Konventionelle Emulgierverfahren basieren bei der Herstellung von ÖI-in-Wasser Emulsionen oder Wasser-in-ÖI-Emulsionen auf einer Tröpfchengrößenzerkleinerung. Bekannt sind Rotor-Stator-Systeme und das Hochdruckhomogenisieren. Beide sind gekennzeichnet durch einen hohen mechanischen Energieeintrag, der vor allem bei empfindlichen Stoffen problematisch ist.

Im Gegensatz dazu werden beim Membranemulgieren Tröpfchen direkt erzeugt, in dem eine disperse Phase durch die Poren einer porösen Membran in eine kontinuierliche Phase gedrückt wird. Die Art der Membran und ihre Porengrößenverteilung nimmt dabei Einfluss auf die erzeugte Emulsion und ihre kennzeichnenden Größen. Die verwendeten Membranen wurden weitgehend für Trennaufgaben (Membranfiltration) entwickelt und eignen sich nur bedingt für das relativ neue Membranemulgieren. Bekannt ist der Einsatz von hydrophilen und hydrophoben Polytetrafluorethylen-Filtern, mikroporösem Glas und poröser Keramik (Homepage der Friedrich-Schiller-Universität Jena, Lehrbereich Lebensmitteltechnologie).

Der Anteil der dispersen Phase in einer Emulsion kann beim bekannten Membranemulgieren nicht beliebig erhöht werden, da hierbei keine oder keine stabile Emulsion mehr erzeugt werden kann. Auch der Einsatz von Emulgatoren, Additiven oder engen Prozessbedingungen führt bei erhöhtem Anteil der dispersen Phase nicht zu den gewünschten Emulsionseigenschaften.

Aus Food Sci. Technol., Int., 2 (1), 43-47, 1996 ist ein sogenanntes Premix-Membranemulgierverfahren bekannt, das als disperse Phase eine Rohemulsion verwendet. Dadurch wird eine Verbesserung des Membranemulgierens erreicht, da der Anteil der dispersen Phase in einem weiteren Bereich variiert werden kann. Auch entfällt ein zusätzlicher Energieeintrag in die kontinuierliche Phase, um Einfluss auf die Tröpfchenbildung an der Membran zu nehmen.

Aus Food Sci. Technol. Int. Tokyo, 4 (2), 164-167, 1998 ist die Verwendung von Polytetrafluorethylen-Membranen bekannt, die sowohl mit hydrophilern als auch hydrophobem Charakter verfügbar sind. Die Porengrößenverteilung dieser Polytetrafluorethylen-Membranen ist breiter als bei porösen Glasmembranen. Die hieraus bekannten Membranen sind für einen universellen Einsatz im großtechnischen Maßstab jedoch nicht geeignet, da hierfür höhere Anforderungen an Stabilität und Durchsatz gestellt werden.

Aufgabe der Erfindung war es demgegenüber, ein Verfahren zum Premix-Membranemulgieren bereitzustellen, welches aus einer Rohemulsion eine feinteilige Emulsion unter der Verwendung von porösen Membranen erzeugt, die einen großtechnischen und universellen Einsatz ermöglichen.

Diese Aufgabe wird durch ein Verfahren zur Herstellung einer feinteiligen Emulsion aus einer Rohemulsion gelöst, wobei die Rohemulsion durch eine poröse Membran gedrückt wird, das dadurch gekennzeichnet ist, dass die poröse Membran aus zwei oder mehreren übereinanderliegenden Schichten aufgebaut ist, die sich durch ihren Porendurchmesser unterscheiden.

Im erfindungsgemäßen Verfahren wird somit eine poröse Membran eingesetzt, die einen asymmetrischen, zwei- oder mehrschichtigem Aufbau aufweist. Hierbei kann es sich um herkömmliche Ultrafiltrations- und Mikrofiltrationsmembranen handeln.

Die mechanische Stabilität der Membran basiert auf einer grobporigen ersten Schicht (Unterstruktur). Sie ist frei tragend und druckstabil, ohne dass hierfür eine Stützvorrichtung erforderlich wäre. Sie dient darüber hinaus als Träger für eine oder mehrere weitere Schichten. Der grobporigen ersten Schicht liegt eine feinporige zweite Schicht gegenüber, die dünner ist als die erste Schicht und die als Dispergierschicht bezeichnet werden kann. Zwischen beiden Schichten können weitere Schichten angeordnet sein, deren Porendurchmesser bevorzugt zwischen dem der Unterstruktur und der Dispergierschicht liegen. Die poröse Membran ist somit bevorzugt aus einer ersten grobporigen Schicht und einer oder mehreren darüberliegenden, gegenüber der ersten Schicht dünneren Schichten mit gegenüber der ersten Schicht geringerem Porendurchmesser gebildet.

Bevorzugt sind auf der ersten grobporigen Schicht mehrere Schichten aufgebracht, deren Porendurchmesser mit zunehmendem Abstand von der ersten Schicht abnimmt.

Ein Verstopfen der Membran wird durch einen solchen asymmetrischen Aufbau weitgehend verhindert.

Es ist möglich, die erfindungsgemäße Membran mit asymmetrischem Aufbau ausgehend von einer symmetrischen Membran herzustellen, indem auf die symmetrische Membran Suspensionen aufgeschlämmt werden. Dieses Verfahren erlaubt den Aufbau von Schichten definierter Porengröße und -verteilung sowie einstellbarer Schichthöhe.

Der Porendurchmesser der grobporigen ersten Schicht der Membran liegt vorteilhaft im Bereich zwischen 1 und 20 µm und ihre Dicke im Bereich von 0,1 bis 10 mm.

Der Porendurchmesser der Dispergierschicht der Membran, der in einem direkten Zusammenhang zu dem erzielten Tröpfchendurchmesser der dispersen Phase in der Feinemulsion und der Tröpfchengrößenverteilung steht, liegt bevorzugt in einem Bereich von 0,01 bis 5 µm. Die Dicke der Dispergierschicht liegt vorteilhaft im Bereich zwischen 1 und 200 µm.

Ein besonders geeigneter Porendurchmesser der Unterstruktur liegt in der gleichen Größenordnung wie der Tröpfchendurchmesser der dispersen Phase der Rohemulsion.

Das Verfahren beinhaltet das Bereitstellen einer Rohemulsion, welche bevorzugt in einem Rührkessel oder einer Mischstrecke erzeugt wird. Als Rohemulsion wird eine Emulsion bezeichnet, in der die Bestandteile der Emulsion, d.h. der Dispersion aus zwei miteinander nicht mischbaren flüssigen Phasen, eine grobe erste Durchmischung erfahren haben.

Demgegenüber wird als Feinemulsion vorliegend eine Emulsion verstanden, deren mittlerer Tröpfchendurchmesser im Bereich von 50 nm bis 100 µm, bevorzugt im Bereich von 100 nm bis 50 µm, liegt. Die Tröpfchen können mittels Laserlichtbeugung (zum Beispiel mit einem Malvern Mastersizer 2000 oder Beckmann-Coulter LS 13320) und/oder mittels dynamischer Lichtstreuung, beispielsweise Photonenkorrelationsspektroskopie, gemessen werden.

Die Prozesstemperaturen beim erfindungsgemäßen Verfahren sind grundsätzlich nicht eingeschränkt. Bevorzugt liegen sie zwischen 0°C und 500°C.

Der aufzubringende Druck, um die Rohemulsion durch die poröse Membran zu drücken, wird insbesondere mittels einer Pumpe Gasdruck oder durch hydrostatische Höhe erzeugt. Die transmembrane Druckdifferenz zwischen Feed- und Produktseite, welche auf den Tröpfchendurchmesser und die Tröpfchengrößenverteilung Einfluss nimmt, liegt zwischen 0,1 bar und 1000 bar, bevorzugt zwischen 0,5 bar und 100 bar, besonders bevorzugt zwischen 1 und 50 bar.

Die poröse Membran kann in unterschiedlichsten Geometrien eingesetzt werden. Beispielsweise sind Flachgeometrien, Rohrgeometrien mit einer innen oder außen aufgebrachten feinporigen Dispergierschicht und Multikanalgeometrien mit mehreren Rohrgeometrien integriert in einer Einheit, sowie Kapillar- oder Wickelgeometrien möglich. Besonders bevorzugt weist die poröse Membran eine rohrförmige Geometrie mit innenliegender oder außenliegender grobporiger erster Schicht auf oder eine ebene Geometrie auf. Dabei sind druckstabile selbsttragende Membranstrukturen bevorzugt, die ohne zusätzliche Stützelemente eine ausreichende Druckstabilität auch bei hohen transmembranen Druckdifferenzen und Durchsätzen im Industriemaßstab gewährleisten. Stützvorrichtungen aus porösen Materialien könnten zur Koaleszenz und damit zu einer Verschlechterung der Feindispergierung führen.

Die poröse Membran wird in entsprechende Druckgehäuse mit einer Trennung von Feed- und Produktseite angeordnet.

Die zwei oder mehreren Schichten der porösen Membran können aus verschiedenen oder aus gleichen Materialien gebildet sein.

Die zwei oder mehreren Schichten können aus verschiedenen oder aus gleichen Materialien gebildet sein.

Je nach stoffspezifischen Anforderungen der zu lösenden Emulgieraufgabe können unterschiedliche Materialien eingesetzt werden. Bevorzugt sind anorganische Werkstoffe, wobei der anorganische Werkstoff insbesondere ein keramischer Werkstoff, ausgewählt aus der Gruppe Aluminiumoxid, Titandioxid, Zirkoniumdioxid, Zirkoniumnitrit oder Mischungen hiervon, Kohlenstoff, Glas, ein Metall oder eine Metalllegierung ist.

Auch die oberflächenaktiven Eigenschaften der eingesetzten asymmetrischen Membran können vorteilhaft an die stoffspezifischen Anforderungen der jeweils zu lösenden Emulgieraufgabe angepasst werden: So werden zur Herstellung von ÖI-in-Wasser-Emulsionen bevorzugt hydrophile oder hydrophilierte Membranen eingesetzt, zur Herstellung von Wasser-in-ÖI-Emulsionen eher hydrophobe Membranen.

Ein unerwarteter und vorteilhafter Effekt auf den erzielbaren Durchsatz, auf die erzielte Tröpfchengröße sowie deren Verteilung, ergibt sich durch eine geeignete Durchströmungsrichtung und eine geeignete Wahl des Porendurchmessers in der ersten grobporigen Schicht (Unterstruktur) und den gegebenenfalls folgenden Zwischenschichten. Liegt die Feedseite auf der Seite der Unterstruktur, d.h. durchströmt die Rohemulsion erst die Unterstruktur und anschließend die feinporige Dispergierschicht, d.h. wird die Rohemulsion von der Seite der ersten grobporigen Schicht durch die poröse Membran gedrückt, wird ein vielfach höherer Durchsatz bei gleichzeitig engerer Tröpfchengrößenverteilung erzielt als dies in umgekehrten Richtung, d.h. von fein nach grob, möglich ist.

Das vorliegende Verfahren eignet sich für eine breite Vielfalt von industriell relevanten Emulsionen und Mikroemulsionen, insbesondere bei scherempfindlichen und temperaturempfindlichen Bestandteilen. Typischerweise für Öl-in-Wasser-Emulsionen, bei denen Öle, organische und anorganische Schmelzen in wässriger Lösung dispergiert werden. Wasser-in-Öl Emulsionen können aus wässrigen Lösungen, Säuren, Laugen, Dispersionen, Lösemitteln, Monomeren bestehen. Das Einsatzgebiet ist breit gefächert, beispielsweise in der pharmazeutischen Industrie für Wirkstoffe, in Salben und in der Lebensmittelindustrie, wobei der Erhalt der Bioaktivität der Inhaltsstoffe im Vordergrund steht.

Eine Reinigung der porösen Emulgiereinheit erfolgt ohne großen Aufwand, da die Membranen mit organischen oder anorganischen Lösungsmitteln und/oder chemisch, beispielsweise mit Säuren, Basen, Oxidationsmitteln oder Reduktionsmitteln im eingebauten Zustand gereinigt werden kann.

Es wurde somit gefunden, dass beim Premix-Membranemulgieren der Membran eine besondere Bedeutung zukommt, da sie einerseits Einfluss auf die Prozessparameter nimmt und andererseits die erzielten Kenngrößen in der Feinemulsion festlegt. Es wurde festgestellt, dass eine Erhöhung des Flusses der Rohemulsion durch die Membran eine Reduzierung der mittleren Tröpfchengröße und der Tröpfchengrößenverteilung mit sich bringt. Der universelle Charakter der erfindungsgemäßen Membrane, d.h. ihre variantenreichen Strukturen, chemischen Eigenschaften und Oberflächeneigenschaften ermöglichen einen Einsatz auch im Industriemaßstab für die unterschiedlichsten Emulgieraufgaben.

Der Einsatz einer asymmetrisch aufgebauten Membran mit zwei oder mehreren Schichten ermöglicht große Membranflächen und hohe Druckdifferenzen beim Premix-Membranemulgieren im großtechnischen Einsatz. Somit können große Membranflächen und hohe Durchflussraten realisiert werden. Die Standzeiten erhöhen sich.

Die Erfindung wird im Folgenden anhand einer Zeichnung und eines Ausführungsbeispiels näher erläutert.

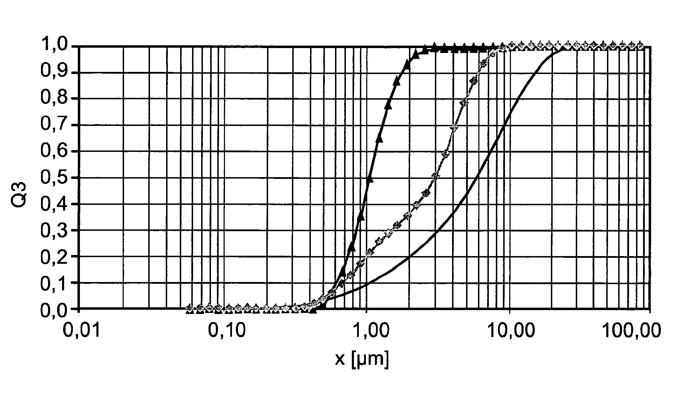

In der Zeichnung zeigt Figur 1 die graphische Darstellung der Tröpfchengrößenverteilung einer Feinemulsion bei unterschiedlicher Durchströmungsrichtung durch eine poröse Membran mit zwei Schichten unterschiedlicher Porosität.

### Beispiel

Untersucht wurde der Einfluss der Anströmrichtung einer porösen Membran auf den Durchsatz und die erzielte Partikelgrößenverteilung.

Hierfür wurde eine scheibenförmige flache UF-Membran der Fa. Inocerminc GmbH aus α-Aluminiumoxid, mit folgendem Aufbau eingesetzt:

Eine erste grobporige Schicht (Unterstruktur) mit einer Dicke von ca. 1 mm und einem mittleren Porendurchmesser von 3 µm, einer gegenüberliegenden feinporigen Schicht (Dispergierschicht) mit einer Dicke von ca. 20 µm und einem mittleren Porendurchmesser von 60 nm und zwei Zwischenschichten mit einer Dicke von ca. je 20 µm und steigendem Porendurchmesser.

Durch diese poröse Membran wurde eine dispergierte Rohemulsion aus Sojaöl, Lutensol® TO 10 (2 Gew.-%) und Wasser (Dispersphasenanteil 10 Gew.-%) bei 30°C und 1 bis 5 bar Differenzdruck gedrückt.

Die Rohemulsion wurde zum einen von der Seite der grobporigen Schicht durch die poröse Membran gedrückt, wobei ein Durchsatz von 186 kg/m²/h/bar erreicht wurde und zum anderen von der Seite der feinen Dispergierschicht, wobei ein nahezu 100-fach kleinerer Durchsatz, von nur 2,1 kg/m²/h/bar, gemessen wurde.

Der Versuch zeigt somit, dass bei einer Durchströmungsrichtung von grob nach fein ein nahezu 100-fach höherer Durchsatz gegenüber der umgekehrten Durchströmungsrichtung erzielt wurde.

Darüber hinaus wurde für beide Durchströmungsrichtungen jeweils die Partikelgrößenverteilung durch Laserbeugung mit einem Gerät Malvern Mastersizer S gemessen. Die Versuchsergebnisse sind in Figur 1 in einem halblogarithmischen Diagramm dargestellt, wobei auf der Abszisse der Tröpfchendurchmesser x in Mikrometern und auf der Ordinate die dimensionslose Massen-Summenverteilung Q3 dargestellt ist. Kurve I zeigt die Partikelgrößenverteilung für die Durchströmungsrichtung von grob nach fein und Kurve II die wesentlich breitere Partikelgrößenverteilung für die Durchströmungsrichtung von fein nach grob.

## Patentansprüche

1. Verfahren zur Herstellung einer feinteiligen Emulsion aus einer Rohemulsion, wobei die Rohemulsion durch eine poröse Membran gedrückt wird, **dadurch gekennzeichnet, dass** die poröse Membran aus zwei oder mehreren übereinanderliegenden Schichten aufgebaut ist, die sich durch ihren Porendurchmesser unterscheiden.

2. Verfahren nach Anspruch 1, **dadurch gekennzeichnet, dass** die zwei oder mehreren Schichten der porösen Membran aus unterschiedlichen Materialien aufgebaut sind.

3. Verfahren nach Anspruch 1 oder 2, **dadurch gekennzeichnet, dass** die poröse Membran aus einer ersten grobporigen Schicht und einer oder mehreren darüberliegenden, gegenüber der ersten Schicht dünneren Schichten mit gegenüber der ersten Schicht geringerem Porendurchmesser gebildet ist.

4. Verfahren nach Anspruch 3, **dadurch gekennzeichnet, dass** auf der ersten grobporigen Schicht mehrere Schichten aufgebracht sind, deren Porendurchmesser mit zunehmendem Abstand von der ersten Schicht abnimmt.

5. Verfahren nach einem der Ansprüche 1 bis 4, **dadurch gekennzeichnet, dass** die Rohemulsion von der Seite der ersten grobporigen Schicht durch die poröse Membran gedrückt wird.

6. Verfahren nach einem der Ansprüche 1 bis 5, **dadurch gekennzeichnet, dass** die disperse Phase der Rohemulsion einen Tröpfchendurchmesser in der Größenordnung des Porendurchmessers der ersten grobporigen Schicht der porösen Membran aufweist.

7. Verfahren nach einem der Ansprüche 1 bis 6, **dadurch gekennzeichnet, dass** die poröse Membran eine rohrförmige Geometrie mit innen liegender oder außen liegender grobporiger erster Schicht oder eine ebene Geometrie aufweist.

8. Verfahren nach einem der Ansprüche 1 bis 7, **dadurch gekennzeichnet, dass** die poröse Membran aus einem anorganischen Werkstoff oder aus mehreren unterschiedlichen anorganischen Werkstoffen gebildet ist.

9. Verfahren nach Anspruch 8, **dadurch gekennzeichnet, dass** der anorganische Werkstoff ein keramischer Werkstoff, ausgewählt aus der Gruppe Aluminiumoxid, Titandioxid, Zirkoniumdioxid, Zirkoniumnitrid oder Mischungen hiervor, Kohlenstoff, Glas, ein Metall oder eine Metalllegierung ist.

10. Verfahren nach einem der Ansprüche 1 bis 9, **dadurch gekennzeichnet, dass** der Druckaufbau, der erforderlich ist, um die Rohemulsion durch die poröse Membran zu drücken, mittels einer Pumpe, durch Gasdruck oder hydrostatische Höhe erzeugt wird.
